**Europäisches Patentamt**

**European Patent Office**

**Office européen des brevets**

(19)

(11) Veröffentlichungsnummer: **0 162 019**
**A1**

(12) # EUROPÄISCHE PATENTANMELDUNG

(21) Anmeldenummer: **85810224.7**

(22) Anmeldetag: **10.05.85**

(51) Int. Cl.⁴: **A 61 F 9/06**

(30) Priorität: **15.05.84 CH 2386/84**

(43) Veröffentlichungstag der Anmeldung: **21.11.85**
**Patentblatt 85/47**

(84) Benannte Vertragsstaaten: **AT BE DE FR GB IT LU NL SE**

(71) Anmelder: **Huber, Peter, Badenerstrasse 90, CH-8952 Schlieren (CH)**

(72) Erfinder: **Huber, Peter, Badenerstrasse 90, CH-8952 Schlieren (CH)**

(74) Vertreter: **Rebmann, John A., Rebmann-Kupfer & Co., Patentanwaltsbureau Augustiner-Glockengasse 18, CH-8022 Zürich 1 (CH)**

(54) **Blendschutz-Vorrichtung für Schmelzschweissungen.**

(57) Um den Blendschutzwirkungsgrad beim Schmelz-schweissen rasch und leicht zu ändern oder beschädigte Blendschutzteile zu ersetzen, ist am Schild (1) eine lösbare Kassette (2) vorgesehen, welche eine durch ein Betätigungs-organ (16) verstellbar in einem Schieber (11) angeordnete Blendschutzscheibe (10) aufweist. Die Kassette (2) kann an einem flachen Mittelteil (1') eines Schildes, der schräg ge-richtete Randteile (1'') besitzt, angeordnet sein.

**EP 0 162 019 A1**

ACTORUM AG

0162019

## Blendschutz-Vorrichtung für Schmelzschweissungen

Zum Schutz der Augen und des Gesichtes werden beim Schmelzschweissen, wie Gas- und Lichtbogenschweissen, in der Hand zu tragende Schilder, auf den Kopf zu setzende Helme und Brillen verwendet, welche mit einer verdunkelbaren Fenster-Oeffnung versehen sind. Die Verdunkelung wird vermittels einer entsprechend ausgebildeten Scheibe herbeigeführt, welche sich durch Verschiebung oder Verdrehung in oder ausser Blendschutzstellung bringen lässt. Bekannt ist die Verwendung von zueinander verstellbaren Polaroid-Gläsern, mittels welchen die Sicht durch die Fenster-Oeffnung verändert werden kann. Die Verstellung der eingefassten Gläser erfolgt manuell oder mechanisch und das Ersetzen ist umständlich und zeitraubend. Die Ersetzung der Gläser erfolgt jeweils bei Beschädigungen oder gewünschter Veränderung der Verdunkelungswirkung.

Die Erfindung betrifft eine Blendschutz-Vorrichtung für Schmelzschweissungen nach dem Oberbegriff des Patentanspruches 1. Derselben liegt die Aufgabe zugrunde die Durchsichtseigenschaften der Gläser leicht sowie rasch verändern, als auch bei Beschädigung auszuwechseln und ersetzen zu können.

Erfindungsgemäss wird diese Aufgabe durch die im Kennzeichen des Patentanspruches 1 definierten Merkmale gelöst. Weitere vorteilhafte Ausbildungen der Erfindung sind den abhängigen Patentansprüchen 2 bis 10 entnehmbar.

Nachfolgend ist der Erfindungsgegenstand an Ausführungsbeispielen in der Zeichnung erläutert. Es zeigen :

Fig. 1 eine Ansicht der Blendschutz-Vorrichtung von hinten,

0162019

Fig. 2   eine SEITENANSICHT DERSELBEN?

Fig. 3   eine Ansicht von vorn,

Fig. 4   einen Schnitt nach der Linie IV-IV

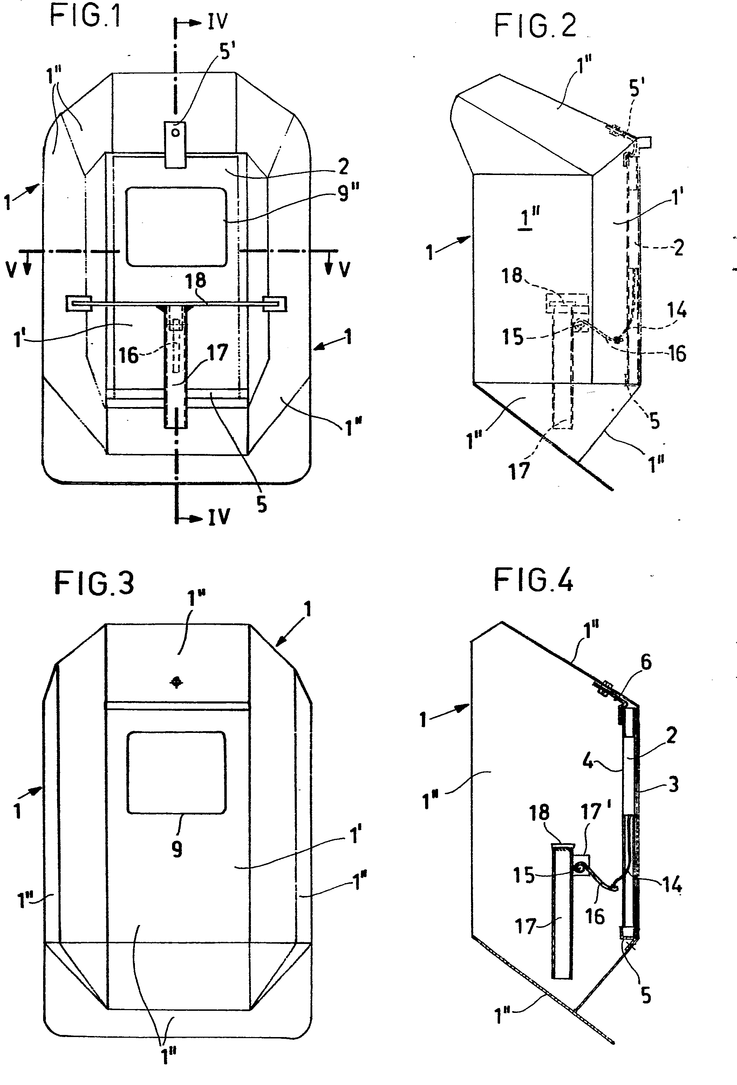

         in Fig. 1,

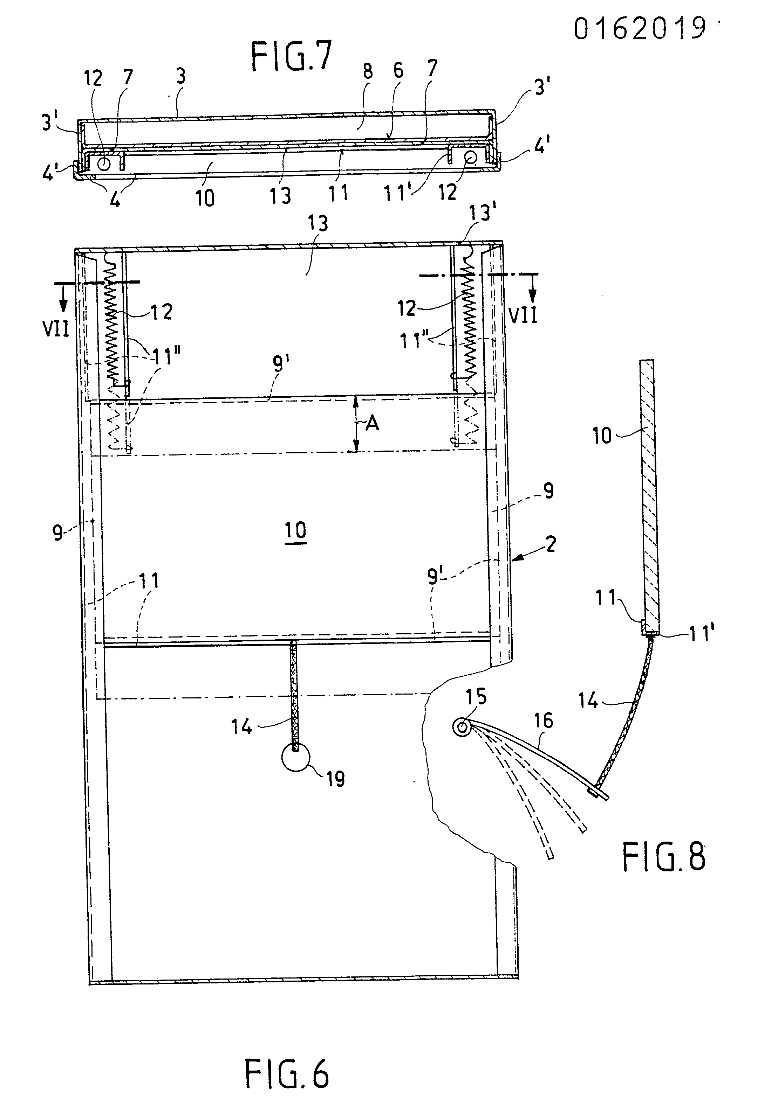

Fig. 5   einen Schnitt nach der Linie V-V
         in Fig. 1,

Fig. 6   die Kassette von hinten in Breitseitenansicht ohne hintere Blechtafel, in
         grösserem Massstab,

Fig. 7   einen Querschnitt nach der Linie VII-VII
         in Fig. 6,

Fig. 8   schematisch die Blendschutzscheibe mit
         dem Verstellhebel in einem ersten Ausführungsbeispiel,

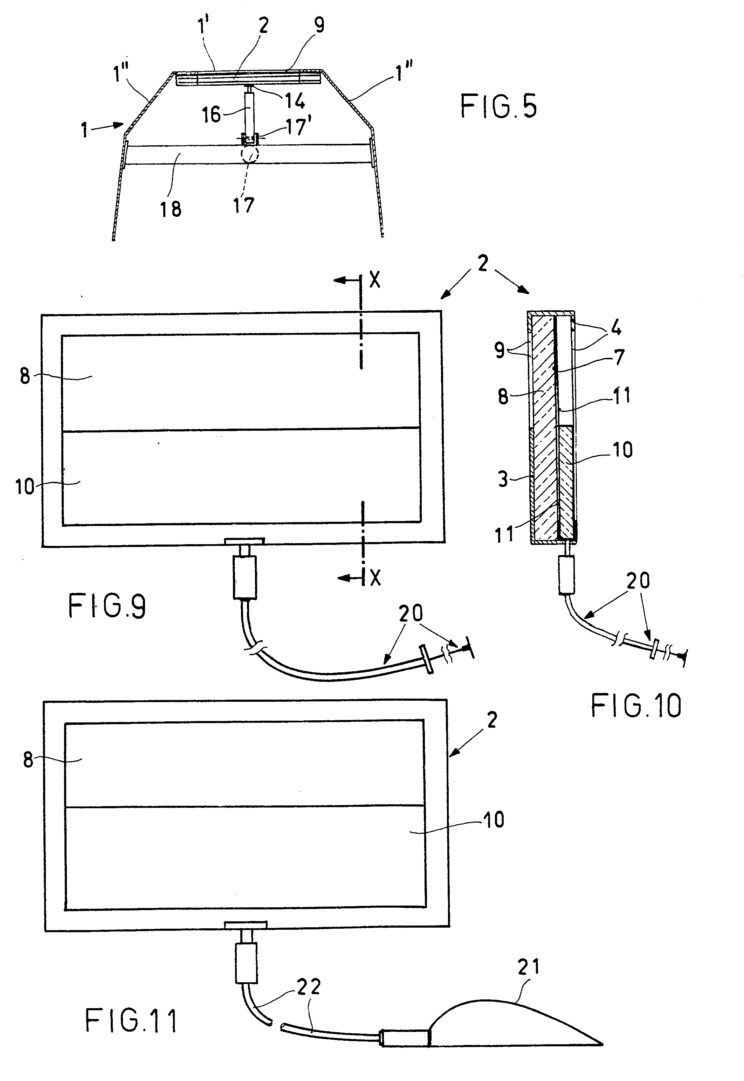

Fig. 9   eine Variante der Kassette in einer
         Breitseitenansicht,

Fig. 10  einen Schnitt nach der Linie X-X in
         Fig. 9 und

Fig. 11  eine zweite Variante der Kassette
         ebenfalls in einer Breitseitenansicht.

1 bezeichnet einen wannenartigen Schutzschild mit eckigen
Umrissen aus Metall oder einem anderen geeigneten Werkstoff.  Der Schild 1 besitzt einen flachen Mittelteil 1'
und schräg zu diesem stehende Randteile 1".  Die letzteren
bezwecken die beim Schweissen entstehenden Funkenwürfe
seitwärts nach hinten abzulenken.  2 ist eine dem Mittelteil 1' hintenseits anliegende und lösbar festgelegte, aus
Blech oder einem anderen geeigneten Werkstoff gefertigte Kassette.  Die gehäuseartige, rechteckige Kassette 2
ist durch eine vordere Tafel 3 und eine hintere Tafel 4
(Fig. 7) abgeschlossen.  Die abgewinkelten Ränder 4' der
hinteren Tafel 4 übergreifen passend an drei Seiten ebenfalls abgewinkelte Ränder 3' der vorderen Tafel 3 (Fig.8).

Die Kassette 2 ist unten in ein am Schild 1 festgemachtes U-Profil 5 teilweise eingesteckt und oben durch eine verschwenkbare, federnde Klammer 5' gehaltert (Fig. 1, 2, 4). Im Innenraum der Kassette 2 zwischen den Tafeln 3, 4 befindet sich eine Trennwand 6 sowie eine Schiebeführungstafel 7. 8 ist die zwischen den Teilen 3, 6 liegende klare Sichtscheibe. Die Sichtscheibe 8 befindet sich hinter der Oeffnung 9 in der Tafel 3 und ist durch Klebung oder Klemmung zwischen den Teilen 3, 6 festgelegt. In der Schiebeführungstafel 7 ist ein in der Höhenrichtung verschiebbarer, dreischenkeliger, rahmenartiger Schieber 11 angeordnet, welcher eine blaue oder braune Blendschutzscheibe 10 trägt. Die Schiebeführungstafel 7 ist mit einer Oeffnung 9' versehen, in deren Bereich die im Schieber 11 eingesetzte Blendschutzscheibe 10 sich befindet. Die Blendschutzscheibe 10 liegt mit der unteren Randseite einem in der Querrichtung verlaufenden, durch Abkröpfung gebildeten Stützteil 11' des Schiebers 11 auf. Der Schieber 11 besitzt an den Höhenseiten oben U-förmige, aufwärts verlaufende Schenkel 11". 12 bezeichnen im Raum zwischen diesen Schenkeln 11" eingefügte Schraubenfedern, deren obere Enden am zugekehrten, abgekröpften Rand 13' der Schiebeführungstafel 7, die unteren Enden dagegen an den Schenkeln 11" hängen.

Die Schraubenfedern 12 bezwecken den Schieber 11 jeweils in die in Fig. 6 gezeigte Normalstellung zu ziehen. In Fig. 6 sind gestrichelt die ausgespannten Schraubenfedern 12 gezeigt, nachdem der Schieber 11 mit der Blendschutzscheibe 10 nach unten gezogen ist, in welcher die Sichtscheibe 8 von hinten gesehen nur teilweise überdeckt ist.

An den Stützteil 11' des Schiebers 11, welchem die Blendschutzscheibe 10 aufliegt, ist ein biegsames Drahtseilstück 14 angeschlossen, das mit dem um Achsbolzen 15

- 4 -

0162019

verschwenkbaren, einarmigen Betätigungshebel 16 verbunden ist. Der Achsbolzen 15 ist im Ansatz 17' des Handgriffes 17 gelagert. Der Handgriff 17, welcher sich im unteren Teil des Schildes 1 befindet und in der Höhenrichtung desselben verläuft, ist an einer Traverse 18 im Schild 1 festgelegt, welche zwei einander quer gegenüberliegende Randteile 1" des letzteren miteinander verbindet. Das Drahtseilstück 14, welches den Betätigungshebel 16 mit dem Stützteil 11' des Schiebers 11 verbindet, ist durch ein in Fig. 6 gestrichelt angedeutetes Loch 19 in der hinteren Tafel 4 geführt.

Das Auf- und Abwärtsverstellen des Schiebers 11 mit der Blendschutzscheibe 10 kann auch durch ein anderes Betätigungsorgan bewirkt sein, wie einem an sich bekannten, bei Photoapparaten verwendeten Drahtauslöser 20. In den Fig. 9 und 10 ist der Drahtauslöser 20 schematisch dargestellt. Die Tafel 4 besitzt ebenfalls eine Oeffnung 9', die ungefähr so gross ist wie die Oeffnung 9 in der vorderen Tafel 3. Zwischen den Schenkeln 11" des Schiebers 11 und der Oberseite der Blendschutzscheibe 10 befindet sich ein Zwischenraum. Die Oeffnungen 9, 9' liegen miteinander auf gleicher Höhe.

Das Verstellen bzw. Verschieben der Blendschutzscheibe 10 lässt sich auch durch Luftdruck mittels einer an sich bekannten Luftpumpe 21 vornehmen, wie in Fig. 11 angedeutet. Der Luftdruck wird durch den Schlauch 22 dem Schieber 11 mit deren Blendschutzscheibe 10 zugeführt.

Die Blendschutzscheibe 10 befindet sich in Normallage auf der Höhe der Oeffnung 9 und wird durch den Zug der Schraubenfedern 12 in diese hochgezogen und gehalten. Bei Verschwenkungen des Betätigungshebels 16 (Fig. 8), wie die gestrichelten Linien zeigen, wird die Blendschutzscheibe 10 mehr oder weniger nach unten gezogen und überdeckt, von hinten gesehen, die Sichtscheibe 8 nur noch teilweise. Die entstandene schmale Klarsicht-Zone ist durch den Doppelpfeil A in Fig. 6 erkenntlich gemacht, diese hat zweckmässig eine Breite von ca. 2-4 cm. Die Klarsicht-Zone ist bekanntlich zur Prüfung der Schweissstellen unerlässlich und die Weite derselben kann in gewissem Ausmass wahlweise grösser oder kleiner sein. Das zeitraubende Wegschwenken des Schildes, um die Schweissstellen für den Schweisser sichtbar zu machen und zu prüfen erübrigt sich, was die Arbeiten wesentlich erleichtert sowie beschleunigt.

Bei einem Drahtauslöser nach Fig. 9 und 10 hält in bekannter Art ein nicht besonders gezeichnetes Arretierelement, wie Schraube, den zugehörigen Draht in mit der Blendschutzscheibe hochgedrückter und angepasster Stellung, in welcher die Oeffnung 9 abgeschlossen ist.

Das Verstellen der Blendschutzscheibe 10 könnte selbstredend auch durch andere Mittel, welche in der Technik bekannt sind oder noch bekannt werden, herbeigeführt sein, wie beispielsweise durch elektromagnetische Kraft, Ultraschall, Laser usw. Das Prinzip einer an einem Träger bzw. Schild 1 leicht lösbar angeordneten Kassette 2, die eine verstellbare Blendschutzscheibe 10 aufweist, ist bei entsprechender Gestaltung auch für Helme sowie Brillen denkbar.

- 1 -

0162019

Patentansprüche:

1. Blendschutz-Vorrichtung für Schmelzschweissungen,
dadurch gekennzeichnet,
dass eine verstellbare Blendschutzscheibe (10) in
einer an einem Träger (1) lösbar angeordneten
Kassette (2) verstellbar angeordnet ist, wobei die
Blendschutzscheibe sich in einem Schieber (11)
befindet, der mit einem Betätigungsorgan (16) in
Bewegungsverbindung steht.

2. Blendschutz-Vorrichtung nach Patentanspruch 1,
dadurch gekennzeichnet,
dass der Träger (1) ein wannenartiger Schild ist,
der einen flachen Mittelteil (1') und schräg zu
diesem gerichtete Randteile (1") besitzt, wobei
die Kassette (2) im Bereich des Mittelteiles, in
welchem sich die Oeffnung (9) einer vorderen
Tafel (3) befindet, wegnehmbar angeordnet ist.

3. Blendschutz-Vorrichtung nach den Patentansprüchen
1 und 2,
dadurch gekennzeichnet, dass die Blendschutzscheibe
(10) sich in einem Schieber (11) befindet, der in
einer Schieberführung (13) angeordnet ist, und
Mittel (12) vorgesehen sind um den Schieber mit der
Blendschutzscheibe nach Verstellung in die Ausgangsstellung zurückzuholen, in welcher eine vor
dieser angeordnete klare Sichtscheibe (8) überdeckt ist.

4. Blendschutz-Vorrichtung nach den Patentansprüchen
1 bis 3,
dadurch gekennzeichnet,
dass die verschiebbare Blendschutzscheibe (10) mit dem Schieber (11) durch ein Zugorgan (14) mit einem am Schild (1) festgelegten Betätigungsorgan (16) in Bewegungsverbindung steht.

5. Blendschutz-Vorrichtung nach den Patentansprüchen 1 bis 4, dadurch gekennzeichnet, dass das Betätigungsorgan (16) ein an einer am Schild (1) festgelegten Traverse (18) verschwenkbarer Hebel ist.

6. Blendschutz-Vorrichtung nach den Patentansprüchen 1 bis 5, dadurch gekennzeichnet, dass das Betätigungsorgan (16) durch einen Drahtauslöser (20) gebildet ist.

7. Blendschutz-Vorrichtung nach den Patentansprüchen 1 bis 5, dadurch gekennzeichnet, dass das Betätigungsorgan (16) durch eine Luftpumpe gebildet ist.

8. Blendschutz-Vorrichtung nach den Patentansprüchen 1 bis 5, dadurch gekennzeichnet, dass das Betätigungsorgan (16) mittels elektrischer Kraft betrieben ist.

9. Blendschutz-Vorrichtung nach den Patentansprüchen 1 bis 5, dadurch gekennzeichnet, dass das Betätigungsorgan (16) drahtlos betrieben ist.

0162019

10. Blendschutz-Vorrichtung nach den Patentansprüchen
    1 bis 5,
    dadurch gekennzeichnet,
    dass das Betätigungsorgan (16) durch Ultraschall
    betrieben ist.

FIG.1

FIG.2

FIG.3

FIG.4

0162019

FIG.7

FIG.6

FIG.8

0162019

FIG.5

FIG.9

FIG.10

FIG.11

Europäisches
Patentamt

**EUROPÄISCHER RECHERCHENBERICHT**

EP 85 81 0224

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 065 909 (DAVID KWAN & CO. LTD.) <br> * Ansprüche 1-9; Seite 2, Zeilen 5-32; Figuren 1-3 * | 1-5 | A 61 F 9/06 |
| X | US-A-2 339 280 (MADSON) <br> * Spalte 2, Zeilen 63-70; Figuren 1,2,5 * | 1-5 | |
| X | DE-A-2 428 301 (POTT) <br> * Ansprüche 1,4; Seite 3, Zeilen 1-5; Figuren 1,3 * | 1-4,6 | |
| X | DE-U-8 120 184 (SCHNEIDER) <br> * Anspruch 1; Figuren * | 1-3 | |
| A | DD-A- 113 998 (WITTIG et al.) <br> * Anspruch 1; Figuren 3,4 * | 1 | RECHERCHIERTE SACHGEBIETE (Int. Cl.4) |
| A | US-A-3 332 087 (MANZ) <br> * Spalte 2, Zeile 71 - Spalte 3, Zeile 2; Figuren 1-5 * | 1 | A 61 F 9/00 <br> F 16 P 1/00 |
| A | US-A-3 086 213 (CROZAT et al.) <br> * Spalte 2, Zeilen 38-44; Figuren 1,2 * | 1 | |
| A | FR-A- 811 772 (HECK) <br> * Figuren 1,2 * | 1 | |

Der vorliegende Recherchenbericht wurde für alle Patentansprüche erstellt.

| Recherchenort | Abschlußdatum der Recherche | Prüfer |
|---|---|---|
| BERLIN | 16-07-1985 | KANAL P K |